Europäisches Patentamt

European Patent Office

Office européen des brevets

(19)

(11) Publication number: **0 031 625**
**B1**

(12) **EUROPEAN PATENT SPECIFICATION**

(45) Date of publication of patent specification: **20.07.83**

(21) Application number: **80201219.5**

(22) Date of filing: **19.12.80**

(51) Int. Cl.³: **C 07 D 201/08,**
**C 07 D 207/267**

(54) Process for the preparation of a 2-pyrrolidone.

(30) Priority: **21.12.79 NL 7909230**

(43) Date of publication of application:
**08.07.81 Bulletin 81/27**

(45) Publication of the grant of the patent:
**20.07.83 Bulletin 83/29**

(84) Designated Contracting States:
**BE DE FR GB IT NL**

(56) References cited:
**DE - B - 1 014 113**
**FR - A - 2 008 643**
**FR - A - 2 316 228**
**FR - A - 2 387 953**
**FR - A - 2 415 100**
**GB - A - 1 240 151**
**US - A - 3 095 423**
**US - A - 4 036 836**
**US - A - 4 123 438**

(73) Proprietor: **STAMICARBON B.V.**
**Postbus 10**
**NL-6160 MC Geleen (NL)**

(72) Inventor: **Meyer, Peter Johan Nicolaas**
**Burg. Luytenstraat 31**
**NL-6151 GE Munstergeleen (NL)**
Inventor: **Nieuwkamp, Johannes Gerardus Maria**
**Grootveldstraat 11**
**NL-6141 LV Limbricht (NL)**

(74) Representative: **Pinckaers, August René et al,**
**OCTROOIBUREAU DSM Postbus 9**
**NL-6160 MA Geleen (NL)**

Courier Press, Leamington Spa, England

Process for the preparation of a 2-pyrrolidone

The invention relates to a process for the preparation of a 2-pyrrolidone by catalytic hydrogenation of optionally substituted succinonitrile in the liquid phase in the presence of ammonia and treatment of the hydrogenated product obtained with water.

According to DE—B—1014113 it is known to prepare 2-pyrrolidone by hydrogenation of β-cyanopropionicacid esters. Later, according to US patent 3095423, FR—A—2316228, US—A—4036836 and US—A—4123438, it was disclosed to prepare 2-pyrrolidone with a good yield by catalytic hydrogenation of succinonitrile in the liquid phase and treatment of the hydrogenated product obtained with water. FR—A—2008643 discloses a two-step process for the preparation of 2-pyrrolidone, involving a suspended catalyst as well. Another two-step process is described in FR—A—2387953, which process relates to the formation of N-substituted 2-pyrrolidones in the presence of primary amines, again involving liquid phase hydrogenation. The objection to these methods is that the catalyst must, after hydrogenation, be separated off, for instance by filtration, which is a costly activity. According to British Patent Application 2.012.748, this objection can be countered by application of a fixed-bed catalyst. In this embodiment the succinonitrile to be hydrogenated is, dissolved in liquid ammonia, passed over the fixed-bed catalyst and there is therefore no filtration or other method required to separate the catalyst from the reaction mixture.

It has now been found that application of a fixed-bed catalyst may reduce the catalyst activity to such an extent that it is necessary to renew the catalyst. The present invention provides a method in which the hydrogenation of the succinonitrile is carried out with a fixed-bed reactor but in which renewal of the catalyst is not, or is less often necessary.

The process according to the invention for the preparation of a 2-pyrrolidone by hydrogenation of succinonitrile, optionally substituted in the 2- and 3- positions by an alkyl group with 1—4 C atoms in the liquid phase in the presence of ammonia using a fixed-bed catalyst and treatment of the hydrogenated product obtained with water is characterized in that the hydrogenation is interrupted one or more times for regeneration of the catalyst by passing liquid ammonia at a temperature of 75—130°C, subsequently hydrogen at a temperature of 130—350°C and then liquid ammonia again at a temperature of 75—130°C over the catalyst.

In the regeneration according to the invention an inert gas such as nitrogen or hydrogen can also be passed over with the liquid ammonia. Hydrogen is usually used as inert gas because this gas is required both for regeneration after passage of the liquid ammonia and for the hydrogenation.

The quantity of liquid ammonia employed for passage over the catalyst in regeneration and the duration of the passage of the liquid ammonia may be variously chosen. The quantity of liquid ammonia may for instance be varied between 1 and 12 kg per hour per kg catalyst. The required duration is usually between 0.5 and 3 hours. A quantity of liquid ammonia greater than 12 kg per hour per kg catalyst and/or a duration greater than 3 hours may be applied, but this is in no way advantageous.

After liquid ammonia has been passed over the catalyst for the first time in the regeneration according to the invention, hydrogen is passed over, for example a quantity of 500—5000 m³ (calculated at 0°C and 1 bar) per hour per m² cross-sectional area of the catalyst bed for a period of e.g. 2—10 hours. A quantity of hydrogen greater than said 5000 m³ and/or period longer than 10 hours may be applied, but are of no practical significance.

After the regeneration according to the invention, the hydrogenation of the succinonitrile can be continued by a known method. In this hydrogenation, the ratio of the succinonitrile to the liquid-phase ammonia may be varied, for instance between 5 and 250 gram of the succinonitrile per 100 gram ammonia present in the liquid phase. A substituted succinonitrile may also be used as starting compound, for instance a succinonitrile substituted in the 2nd or 3rd position by an alkyl group with 1—4 C atoms.

The hydrogen partial pressure during hydrogenation may also be varied, for instance between 1 and 350 bar. A hydrogen partial pressure of between 50 and 200 bar is preferably applied. The temperature is usually chosen between 40 and 150°C, preferably between 50 and 130°C.

As catalyst for the hydrogenation of the succinonitrile, any hydrogenation catalyst may be employed, as for example nickel, cobalt or palladium, optionally on a suitable carrier such as for instance carbon, aluminium oxide or silicon dioxide. The hydrogenation is usually performed with a nickel-containing catalyst.

A so-called trickle-phase reactor is very suitable for operating the hydrogenation. The solution of the succinonitrile in liquid ammonia then flows under the influence of gravity through the fixed catalyst bed while the hydrogen or the hydrogen-containing gas is passed countercurrently of cocurrently through the catalyst bed. Here, the specific catalyst loading may be variously chosen, for instance between 0.1 and 25 m³ liquid per m³ catalyst per hour.

After the hydrogenation of the succinonitrile, the ammonia may be wholly or partially removed from the reaction mixture obtained because the treatment of the hydrogenated product with water can be effected in the presence or in the absence of ammonia. In this water treatment the temperature may be variously chosen, as in the known method, for instance between 150 and 300°C. The quantity of water, too, may be varied as in the known method, for instance between the stoichiometrically required quantity and 20 mole water per mole succinonitrile.

The method according to the invention is now further elucidated in the following example.

### Example

In a mixer heated to 80°C, 0.2 kg succinonitrile was dissolved in 1.85 kg liquid ammonia at elevated pressure, whereafter the solution obtained was pumped to the top of a vertically positioned metal tubular reactor (length 1.5 metre, internal diameter 2.54 cemtimetre).

The bottom layer in the reactor was 700 millilitre catalyst and the top layer 75 milli-litre inert packing material (protruded metal packing, dimensions 0.16 x 0.16 cm). The catalyst used was a commercially available activated nickel catalyst (50 wt. % nickel on $Al_2O_3$ as carrier), in the form of cylinders with a height of 4.2 mm and a diameter of 4.0 mm. The bulk density of the catalyst was 970 gram per litre.

Simultaneously with the ammoniacal solution, hydrogen was fed to the top of the tubular reactor by means of a compressor at a rate of 1400 l (calculated at 0°C and 1 bar) per hour. The hydrogen partial pressure in the reactor was maintained at 75 bar (total pressure 140 bar). The temperature in the reactor was controlled by means of jacket heating such that the reaction mixture to be discharged had a temperature of 85°C.

The reaction mixture thus obtained was discharged from the bottom of the reactor, subsequently cooled to 30°C and separated at elevated pressure in a separator into liquid and gas. Subsequently, ammonia was removed from the reaction mixture in an expansion vessel operated at atmospheric pressure. The reaction mixture was periodically collected for 2 hours for analysis. From the reaction mixture thus collected, a 2-gram sample was gas-chromatographically analysed, showing that no or substantially no starting compound was present. The remaining quantity of collected reaction mixture was heated together with 200 gram water in a stirred 1-litre autoclave for 1.5 hours at 210°C. After cooling, the quantity of pyrrolidone in the hydrolysed product was determined gas-chromatographically. The periodic analyses showed that in a 150-hour operating period the succinonitrile conversion was 95—100% and the pyrrolidone selectivity 70—75%.

When, after a number of interruptions in the hydrogenation (thrice in a 10-hour period due to a malfunctioning ammonia feed), the conversion dropped to approx. 20% over an 8-hour period, the catalyst was regenerated as follows: liquid $NH_3$ (2 kg per hour) and hydrogen (1000 l, calculated at 0°C and 1 bar, per hour) were passed over the catalyst for 1.5 hours at a temperature of 85°C. The total pressure was 140 bar. Subsequently, hydrogen (1000 l per hour, calculated at 0°C and 1 bar) was passed over the catalyst for 4 hours at a temperature of 175°C. The total pressure was 140 bar. After cooling of the catalyst to 85°C liquid $NH_3$ (2 kg per hour) and hydrogen (1000 l, calculated at 0°C and 1 bar, per hour) were again passed over the catalyst for 2 hours at a total pressure of 140 bar.

The catalyst thus regenerated was employed again for the hydrogenation of succinonitrile by the method described above for a period of 200 hours with the result that the original conversion and selectivity levels were again reached.

### Claim

Process for the preparation of a 2-pyrrolidone by hydrogenation of succinonitrile, optionally substituted in the 2- and 3- positions by an alkyl group with 1—4 C atoms, in the liquid phase in the presence of ammonia using a fixed-bed catalyst and treatment of the hydrogenated product obtained with water, characterized in that the hydrogenation is interrupted one or more times for regeneration of the catalyst by passing liquid ammonia at a temperature of 75—130°C, subsequently hydrogen at a temperature of 130—350°C and then liquid ammonia again at a temperature of 75—130°C over the catalyst.

### Revendication

Procédé de préparation d'une 2-pyrrolidone par hydrogénation de succinonitrile, éventu-ellement substitué dans les positions 2 et 3 par un groupe alcoyle en $C_1$ à $C_4$, en phase liquide en présence d'ammoniaque en utilisant un catalyseur à lit fixé, et traitement du produit hydrogéné obtenu avec de l'eau, caractérisé en ce qu'on interrompt l'hydrogénation une ou plusieurs fois pour la régénération du catalyseur en faisant passer de l'ammoniaque à une température de 75—130°C, puis de l'hydrogène à une température de 130—350°C puis à nouveau de l'ammoniaque à une tempèrature de 75—130°C sur le catalyseur.

### Patentanspruch

Verfahren zur Herstellung eines 2-Pyrroli-dons durch Hydrierung von Bernsteinsäure-nitril, wahlweise in den 2- und 3-Stellungen durch eine Alkylgruppe mit 1—4 C-Atomen substituiert, in der wässrigen Phase in Anwesenheit von Ammoniak unter Verwendung eines Schüttschicht-Katalysators und Behandlung des hydrierten Produkts mit Wasser, dadurch gekennzeichnet, dass die Hydrierung einmal oder mehrmals unterbrochen wird für die Regenerierung des Katalysators durch überleiten von flüssigem Ammoniak bei einer Temperatur von 75—130°C, anschliessend Wasserstoff bei einer Temperatur von 130—350°C und danach wiederum flüssigem Ammoniak bei einer Temperatur von 75—130°C über den Katalysator.